# EUROPEAN PATENT SPECIFICATION

(11) **EP 1 649 743 B1**
(45) Date of publication and mention of the grant of the patent: **08.10.2008**
(21) Application number: 05256545.4
(22) Date of filing: 21.10.2005
(51) Int. Cl.: A01H 4/00, C12N 15/82

(54) **Method of direct regeneration, Shikonin induction in callus and Agrobacterium rhizogenes-mediated genetic transformation of Arnebia hispidissima**
Methode zur direkten Regeneration, Shikonin Induktion im Kallus und genetische Modifizierung von Arnebia hispidissima mit Agrobacterium rhizogenes
Méthode de régénération directe, de l'induction de Shikonin dans le cal et de la modification génétique d'Arnebia hispidissima avec Agrobacterium rhizogenes

(30) Priority: 21.10.2004 IN DE20662004; 14.12.2004 IN DE24832004
(43) Date of publication of application: 26.04.2006
(73) Proprietor: Guru Jambheshwar University, Haryana (IN); Department of Biotechnology, New Delhi-11000 (IN)
(72) Inventor: Chaudhury, Ashok Department of Biotechnology, Haryana (IN); Pal, Minakshi Department of Biotechnology, Haryana (IN)
(74) Representative: Green, Mark Charles

(56) References cited:
- CN-A- 1 117 525
- PATENT ABSTRACTS OF JAPAN vol. 2000, no. 04, 31 August 2000 (2000-08-31) & JP 2000 004702 A (SANEI GEN FFI INC), 11 January 2000 (2000-01-11)
- YU HEE-JU ET AL: "Plant regeneration from callus cultures of Lithospermum erythrorhizon" PLANT CELL REPORTS, vol. 16, no. 5, 1997, pages 261-266, XP002360545 ISSN: 0721-7714
- DATABASE CA CHEMICAL ABSTRACTS SERVICE, COLUMBUS, OHIO, US; 2001, JI, QIAOLING ET AL: "Asexual propagation of Arnebia euchroma johnston and exploration of hereditary stability in regenerated plantlet" XP002360549 retrieved from STN Database accession no. 137:77949 & ZHIWU SHENGLIXUE TONGXUN , 37(6), 499-502 CODEN: CHWSAX; ISSN: 0412-0922, 2001,
- SINGH B ET AL: "Estimation of naphthaquinones from Arnebia hispidissima (Lehm.) DC. In vivo and in vitro. I. Anti-inflammatory screening." PHYTOTHERAPY RESEARCH : PTR. FEB 2004, vol. 18, no. 2, February 2004 (2004-02), pages 154-159, XP002360559 ISSN: 0951-418X
- DATABASE EMBASE ELSEVIER SCIENCE PUBLISHERS, AMSTERDAM, NL; 2004, GE F ET AL: "Studies on highly efficient induction of callus from Arnebia euchroma and rapid proliferation of callus" XP002360550 Database accession no. EMB-2005011825 & CHINESE PHARMACEUTICAL JOURNAL 2004 CHINA, vol. 39, no. 10, 2004, pages 735-737, ISSN: 1001-2494
- DATABASE BIOSIS BIOSCIENCES INFORMATION SERVICE, PHILADELPHIA, PA, US; 1991, DAVYDENKOV V N ET AL: "CULTURED ARNEBIA-EUCHROMA ROYLE JONST. CELLS A NEW SOURCE OF SHIKONIN PRODUCTION" XP002360551 retrieved from BIOSIS Database accession no. PREV199192032344 & KHIMIKO-FARMATSEVTICHESKII ZHURNAL, vol. 25, no. 1, 1991, pages 53-55, ISSN: 0023-1134
- PATENT ABSTRACTS OF JAPAN vol. 012, no. 387 (C-536), 14 October 1988 (1988-10-14) & JP 63 133996 A (LION CORP), 6 June 1988 (1988-06-06)
- MANJKHOLA SUMIT ET AL: "Organogenesis, embryogenesis, and synthetic seed production in Arnebia euchroma - A critically endangered medicinal plant of the Himalaya" IN VITRO CELLULAR & DEVELOPMENTAL BIOLOGY PLANT, vol. 41, no. 3, May 2005 (2005-05), pages 244-248, XP009059371 ISSN: 1054-5476

## Description

### FIELD OF THE INVENTION:

This invention relates to a method of direct plant regeneration, callus production, Shikonin induction and Agrobacterium rhizogenes-mediated genetic transformation in Arnebia species. The invention also provides an in vitro method for induction of Shikonin production in callus cultures of Arnebia hispidissima. The invention also provides a method for production of Hairy Root Culture via Agrobacterium-mediated genetics transformation of Arnebia and induction of Shikonin production.

### BACKGROUND OF THE INVENTION:

Arnebia (family Boraginaceae) is a genus of hispid herbs, mostly confined to Asia with a few species occurring in drier part of North Africa. Seven species are known to occur in India. These include A. benthamii, A. euchroma, A. guttata, A. hispidissima and A. nobitis.

The plant of genus Amebia as well as some other species of Boraginaceae from the genera Lithospermum, Onosma, Echium etc, provides the source of naphthoquinonous red pigments Shikonin. Shikonin has been known since ancient times as a dye used for Silk and Food Industry. At the same time, Shikonin is recognized as a remedy, showing a wide range of medical applications. It possesses antibacterial, antifungal activities and exhibits antiinflammatory and wound healing properties. The anti-allergic, antipyretic, antihydropic effects of Shikonin and its derivatives as well as antineoplastic activities are reported (Terada et. Al. 1990). Most of the species of Boraginxex yield "Ratanjot", which is used as a red dye and also as a medicine to treat a variety of ailments (Khatoon et. Al. 1994 and 2003). Shikonin and its derivatives are predominantly predisposed in the outer surface of roots of Amebia. Shikonin has an applicable pharmacological effect as an antibacterial agent, granulation tissue forming activity and anti-uker activity (Hayashi et. Al 1969; Papageorgiou, 1980). Shikonin content depends upon the age of plant and other environmental factors. Middle aged plant with 6-8 flowering stems, show highest level of Shikonin (Pimenova and Tareeva, 1980). Wild plant species from the Boraginaceae family, accumulating Shikonin fail to provide a sufficient raw material for commercial production, owing to which tissue culture technique becomes an excellent tool for overcoming these short fall in ever-increasing demand worldwide. Arnebia tissue culture was first reported by Davydenkov et. Al (1991). However, no detailed studies have so far been reported on high frequency direct plant regeneration in this important medicinal plant species.

Shikonin biosynthesis has been one of the most intensively studied topics in the area of secondary metabolite production of higher plants (Tabata, 1996), and many of the regulatory factots in the biosynthetic pathway have been identified. The strongest inhibitor of Shikonin biosynthesis is light (Tabata et. Al. 1974; Heide et. Al. 1989) Several cDNAs of dark-expressed genes have been cloned from Lithospermum erythrorhizon by subtractive hybridization (Yazaki et. Al 1998), but neither their function in vivo nor the molecular mechanism of light inhibition is known because no stable transformation system is available and the major constraints with Lithospermum cell culture is that they are genetically unstable and cultured cell tend to produce low yield of secondary metabolites with the passage of time.

The hairy root disease is a pathological syndrome of dicotyledonous plants following wounding and infection with Agrobacterium rhizogenes. The rhizogenicity is conferred upon to plant cells by a fragment of DNA (RiT-DNA), which is transferred from the large root inducing (Ri) plasmid, harboured by the bacterium, to the genome, where it is stably integrated and expressed. Integration of a DNA segment (T-DNA) of pRi into the host genome leads to active proliferation of adventitious roots commonly referred to as Hairy Roots at or near the site of infection. The Rol genes and RiT-DNA induces changes in sensitivity in plant hormone or in the metabolism of plant hormones (Maurel et. Al. 1994; Moritz and Schmulling 1998; Shen et.al. 1988). Furthermore, transformation of plant tissue by infection with Agrobacterium rhizogenes increases the production of certain metabolites (Ermayanti et. Al. 1994; Mano et. Al. 19861 Sim et. al. 1994). If the Agrobacterium rhizogenes containing modified Ri plasmid is used to infect plant cells, it is possible to transfer the target genes to plant tissues and to induce regeneration of transgenic plants. Therefore, Agrobacterium rhizogenes can be used in genetic manipulation of higher plants for improving plant resistance to biotic and biotic stress, quality improvement and potential industrial application. Many reports about genetic transformation of various medicinal plants have been published including Lithospermum erythrorhizon through induction of Hairy Root Cultures. However, Agrobacterium rhizogenes-mediated genetic transformations of Arnebia hispidissima have not been reported so far.

The development and commercialization of medicinal plant-based bio-industries in developing countries is dependent upon the research and development inputs related to bio-processing, extraction, purification and marketing. A number of countries in the west have been fairly successful in utilizing the potential of medicinal plants. In Germany, for example, over 1500 plant species have been processed into medicinal products (Lewington, 1993).

Medicinal plants and their habitats are under severe threat of erosion due to their over exploitation mainly for exports under liberalized economic policy of Government of India. An estimated 1'00 medium-scale herbal drug industries and over 5000 small-scale including cottage level industries are using around 450 species of medicinal plants. Among theses 95% of the raw materials are collected from the wild. Furthermore, about 4 lakhs registered physician of traditional medicine systems also use huge quantity of medicinal plants procured through the crude herbal drug dealers who obtain their supplies mainly from the wild collectors (Tewari, 2000).

Keeping the view the importance of medicinal plant world wide, the present invention was carried out on Arnebia hispidissima belonging to family Boraginaceae, provides the source of naphthoquinonous red pigment Shikonin. Shikonin has been known since ancient times as a dye used for silk and food products. Most of the species of Boraginaceae yield Rataniot, which is used as a red dye and also to treat variety of ailments (Khatoon et. Al. 1994 and 2003). Species of the genus Arnebia have various pharmacological properties such as Shikonin from roots of Lithospermum erythrorhizon, Echium tycopsis, Onosma caucasicum shows antitumor activity. Roots of Arnebia nobilis, Alkana tinctoria and Macrotomia cephalotes and leaf surface constituents of Plagiobothrys arizonicus yield alkanin which at low concentration has immunomodulatory effects and at higher concenstration has suppressive action in granulocyte and lymphocyte systems. It is used in the treatment of Ulcus cruis. Arnebin 1, β,β-dimethylacrylalkannin from roots of Amebia nobilis has anti-cancer property (Anonymus, 1996). A. euchroma, another species represented in the high altitute Himalaya (3000-4200 m) is a hispid erect herb of high medicinal value. The leaves and flowers are collected and dried and water extract serves as a remedy for fevers. The roots are collected for extracting pinkish-reddish dye for dying of milk products like butter, cheese etc. Bioactivity directed fractionation of root extracts of A. euchroma exhibit potent anti human immunodeficiency virus activity HIV (Kashiwada et. Al. 1995) and rubricine, a broad spectrum antimicrobial agent is found to be active against bacteria as well as fungi. Root extracts of the genus Arnebia, have been in use for over 3000 years as a topical wound heating agent in Asia and Europe to treat cuts, burns, bruises and other skin related conditions. It is also reported to eliminate blood heat and promoting circulation of the blood, removing toxic materials and letting eruptions out, promoting diuresis and facilitating defecation. The drug is applied as an external emollient to eczema, abscesses and bums; the oil is also quite effective against diaper rash.

As a result of over exploitation of its rhizomes for medico purposes Arnebia benthamii has been listed in the Indian Red Data Book (Jain and Sastry, 1984).

Apart from reports on taxonomic descriptions, general distribution and uses, detailed investigations have not been undertaken so far, especially with regard to the biology, development of micropropagation protocols and other related aspects of this important medicinal plant species.

Furthermore, JP2000004702 discloses the regeneration of a Boraginacea plant (Lithospermum sp.) from explants, which are sterilized and then incubated in appropriate medium for plant regeneration and growth. Yu Hee-Ju *et al,* 1997, discloses the regeneration of a Lithospermum plant from callus, which was originally obtained from seed. Ji , Qiaoling *et al*, 2001, discloses the regeneration of Arnebia euchroma plantlets from callus. Singh *et al*, 2004, describes a method of estimation of Naphthaquinones from dried roots of mature plants and in vitro grown seed derived callus from Arnebia hispidissima and their anti-inflammatory effect whilst Ge *et al,* 2004, discloses the induction of callus from explants of Arnebia euchroma.

Keeping in view of medicinal importance of the species, it is important to develop protocols for the induction of Shikonin production through tissue culture and mictropropagation techniques from various explants of Arnebia hispidissima. The interest in Hairy Root is due to their ability to grow fast without requiring an external supply of auxins and many times does not require light. They have been found to be fairly stable in metabolite yield due to their genetic stability. Owing to these advantages many of the root derived plant products once not considered feasible for the production by cell culture are being reinvestigated for production using the Hairy Root Culture technology. Many reports about hairy root transformation of various other medicinal plants have been published including Lithospermum erythrorhizon. However, Agrobacterium rhizogenes-mediated genetic transformations of Arnebia hispidissima has not been reported so far.

### OBJECTS OF THE INVENTION:

An object of the present invention is to provide a rapid and efficient method of regeneration of whole plant of Arnebia species preferably Arnebia hispidissima.

Another object of the present invention is to provide a method of high frequency direct plant regeneration of Arnebia hispidissima from shoot tips by supplementing the media with various plant growth regulators.

Another object of the present invention is to provide a method for high frequency direct plant regeneration of Arnebia hispidissima from explants selected from leaf segments, nodal and internodal segments.

Another object of the invention is to provide a method for regeneration of true-to-type planlets of Amebia hispidissima.

Another object of the invention is to provide an optimized media composition for Direct Plant Regeneration of Arnebia hispidissima without callus phase.

Still another object of the invention is to provide method for production of callus cultures from various explants of Arnebia hispidissima.

Another object of the invention is to provide an optimized media composition for callus production of Arnebia hispidissima.

Still another object of the invention is to provide an in vitro method for induction of Shikonin in callus cultures of Arnebia hispidissima.

Another object of the invention is to provide an optimized media composition for induction of Shikonin in callus cultures of Arnebia hispidissima.

Another object of present invention is to provide a method for Agrobacterium rhizogenes-mediated genetic transformation in Arnebia species, preferably Arnebia hispidissima.

Still another object of the invention is to provide a method for co-cultivation for production Hairy Root Culture and induction of Shikonin production in transformed Arnebia species, preferably knebia hispidissima.

### SUMMARY OF THE INVENTION :

According to this invention there is provided a method for the Direct Plant Regeneration of Arnebia hispidissima plant, said method comprising the steps of :
obtaining explants of Arnebia species sterilizing the said explants,
subjecting the said sterilized plants to the step of shoot regeneration by culturing the explants in shoot induction MS medium,
subjecting the said shoots to the step of root generation by culturing the said shoots in a root induction, MS medium,
transferring the said plantlets with shoots and root into the soil for regeneration

In accordance with this invention there is also provided a method for the production of callus and induction of shikonin comprising the steps of :
obtaining explants of Arnebia hispidissima ,
sterilizing the nodal segment explants,
subjecting the sterilized nodal explant to the step of callus production by
culturing in a callus induction MS medium,
cultering the said callus in a shikonin induction MS medium,
extracting shikonin form the said medium.

Further according to this invention there is provided a method for genetic transformation in Arnebia species by Agrobacterium rhizogenes for the production of Shikonin comprising the steps of:
sterilizing the explant of Arnebia species,
inoculating the said sterilized explant in MS medium to form cluster of multiple shoots
preparing LB medium for Agrobacterium culture to obtain single colonies of Agrobacterium rhizogenes,
inoculating said colonies of Agrobacterium rhizogenes in LB medium in incubator,
infecting said shoots of arnebia species with Agrobacterium rhizogenes,
co-cultivating said infected explants in MS medium
subjecting the said cultures to the step of incubation in complete darkness.
treating the infected shoots in a medium supplemented with anti biotic,
subjecting the said shoots to the step of sub culturing in root culture medium
extructing Shikonin derivatives from the roots.

### BRIEF DESCRIPTION OF THE ACCOMPANYING FIGURES :

- Figure 1 :: Explants showing shoot initiation.
- Figure 2 :: Regenerated plantlets showing complete shoot and root initiation.
- Figure 3:: Green callus under light conditions showing no pigmentation development.
- Figure 4 :: Red pigmented callus showing enhanced Shikonin production.
- Figure 5 :: Explants showing Hairy Root Culture initiation and calli formation in vitro.
- Figure 6 :: Hairy Root Culture initiation with appearance of red spots of Shikonin induction.
- Figure 7 :: Second subculture in hormone free MS Semi-Solid medium showing reddish roots turning white.
- Figure 8 :: Induction of Shikonin production in Hairy Root Cultures initiated on the ammonia free RC media.

### DETAILED DESCRIPTION OF THE INVENTION :

The present invention provides an efficient method for high frequency direct plant regeneration of Arnebia species, preferably, Arnebia hispidissima. The present invention also provides a method for high frequency direct plant regeneration from various explants, wherein, the explants are selected from a group consisting of shoot tip, nodal and internodal explants, leaf segment and other suitable explants. The invention provides a method for regeneration wherein the explants are cultured on the MS media containing plant growth regulators.

The invention also provides media composition for shoot regeneration of Arnebia species preferably Arnebia hispidissima.

Further the invention also provides a media composition for callus production from various explants of Arnebia species preferably Arnebia hispidissima.

Further the invention also provides a media composition for Shikonin induction in callus culture of Arnebia species preferably Amebia hispidissima.

The invention further provides an in vitro method for induction of pigment Shikonin in callus cultures of Arnebia hispidissima.

### Establishment of Arnebia hispidissima In vitro Cultures

For high frequency direct plant regeneration of Amebia hispidissima various explants were tested. For regeneration of Arnebia hispidissima field grown plants were obtained from Guru Jambheshwar University of Science and Technology Hisar, Haryana,India. Different types of plants were tested for direct regeneration of plantlets.

The explants selected are :
1) Shoot tips (4-6 mm long) which consist of 10-15 developing leaves surrounding the meristem.
2) Leaf segment : Young leaves were taken from the tip of the main branch.
3) Nodal segments and internodal segments employed were taken from third to sixth node from the tip of the branch.

For shoot tip cultures, bud scales and outer developing leaves were removed and the shoot tip was excised using a sharp, sterile scalpel blade.

Leaf segments were prepared by cutting young leaf into 5-10 mm length. Nodal and internodal segments were prepared by cutting the stem into 5-10 mm length.

All the explants were washed with mild detergent (Teepol) using standard procedures, followed by several rinses in tap water. These were then surface sterilized with 0.1% mercuric chloride (HgCl₂) for 5-7 minutes and, subsequently, washed three times with double distilled water to remove traces of HgCl₂.Explants were placed with the abaxial side down on suitable medium in glass jars with caps.

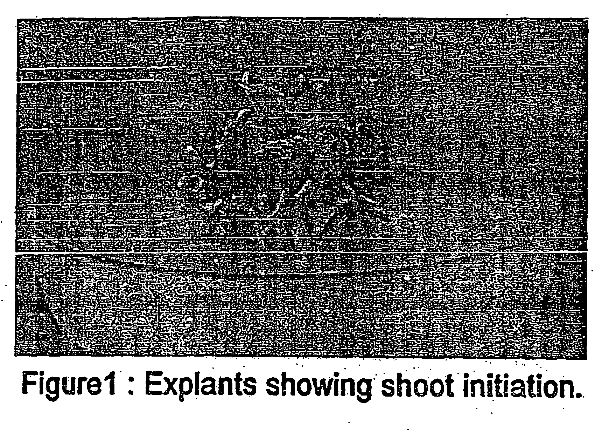

The surface sterilized explants were cultured on MS media supplemented with various hormones for shoot regeneration as given in Table 1. The shoot regeneration from various explants of Arnebia hispidissima using various shoot induction media combination is shown in Table 2. The explants were incubated on media for four weeks for regeneration of multiple shoots (Figure 1). The explants were maintained in a growth chamber with the following conditions, namely, temperature of 25 ±2°C , relative humidity (RH) of 50-60% and 16 hour photoperiod with fluorescent light (1000 µmol M⁻² S-1photosensitivity), followed by a 8h dark period.

The tissue culture MS medium (Murashige and Skoog, 1962) supplemented with BAP (0.25mgl⁻¹), Kinetin (0.25 mg l⁻¹), IAA (0.1 mgl⁻¹) and Casein hydrolysate (CH, 100.0 mgl⁻¹) showed 53.3 and 40 percent shoot regeneration from shoot tip and nodal explants, respectively, as shown in Table-2.

The tissue culture MS medium supplemented with BAP (0.5 mgl⁻¹), Kinetin (1.0 mgl⁻¹) IAA (0.1 mgl⁻¹) and CH(100.0 mgl⁻¹) showed 66 and 40 percent shoot regeneration from shoot tip and nodal explants respectively (Table 2), Shoot tips cultured on MS medium supplemented with BAP (0.25 mgl⁻¹), Kinetin (0.5 mgl⁻¹), IAA(0.1 mgl⁻¹) produced 93.3 percent shoot regeneration with multiple shoots as shown in Table 2. This media combination also resulted in more than 60 percent shoot regeneration from nodal segments. This media combination produced multiple shoots with short hairs mixed with bristly bulbous based hairs. Leaves of length 1.5 - 5.0 cm were linear and lanceolate and developed directly from the explants without callus production. Thus, the ideal combination of BAP, Kinetin and IAA is identified for effective and high frequency shoot regeneration from the various explants. MS media containing BAP alone was ineffective in inducing shoot proliferation. Generally, shoot tip explants were more responsive than nodal explants. Internodal explants hardly showed any shoot regeneration even under various media combination and culture room conditions employed.

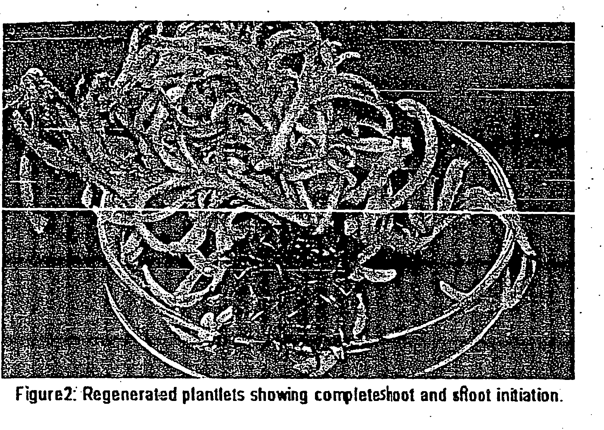

Further, the shoots were allowed to elongate in the same medium to a length of 1.5 cm to 2 cm and were cultured on root induction medium for rooting. Different rooting media combinations were tested for root induction and these combinations are shown in Table 3. Among the various auxins screened (IBA) was effective in initiating roots (Figure 2). The ideal media combination for root inductions is MS medium supplemented with IBA (2 mgl⁻¹). The in vitro rooted shoots resumed growth after a short period of acclimatization and resulted in plantlets which were transferred to the soil after hardening. These regenerated plantlets were successfully established in soil and appeared phenotypically similar to wild type plants. Another interesting observation is the in vitro flowering of tissue culture raised plantlets when these plantlets are maintained for long periods in tissue culture media.

### Regeneration of Plants fran Callus of Arnebia hispidissima

Various explants, namely, leaf segments, internodal segments and roots were cultured on different media combinations given below :
1) MS medium along
2) MS medium supplemented with 2,4-Dichloro-phennoxyacetic acid (2,4-D, 0.25 0.5, 1.0, 1.5, 2.0 mgl⁻¹)
3) MS medium containing 2,4-D (1.0 mgl⁻¹) in combination vrith 6-Benzylaminopurine (BAP 0.25,0.5.1.0,1.5,2.0 mgl⁻¹)
4) MS medium containing Kinetin (KIN, 0.25, 0.5 1.0, 1.5, 2.0 mgl⁻¹) and 2, 4-D (1.0 mgl⁻¹)
5) MS medium containing Indole-3-aceticacid (IAA, 0.25, 0.5, 1.0, 2.0 mgl⁻¹) alone or in combination with BAP (0.25, 0.5, 1.0 mgl⁻¹) and Kinetin (0.25, 0.5, 1.0 mgl⁻¹)

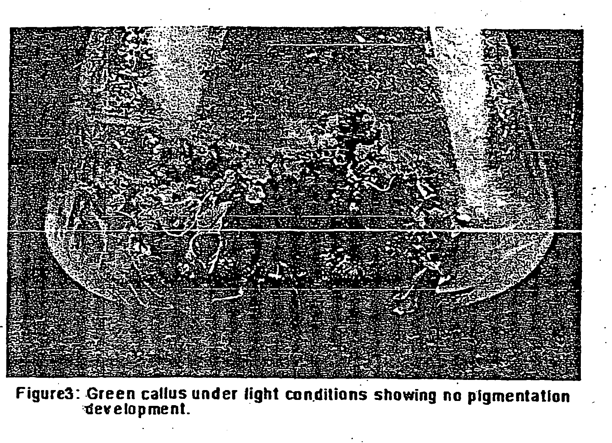

The tissue culture MS media supplemented with Kinetin (0.5 mgl⁻¹) and 2,4-D (1.0 mgl⁻¹) resulted in the highest production of 86.7% callus from nodal explants. Calli was obtained with other media combinations also as shown in Table 4. Calli obtained with various explants were green under white light and yellowish white in the dark conditions (Figure 3). Callus cultures failed to produce any pigment even after several sub-cultures in the media combinations shown in Table 4.

The callus obtained were sub cultured on various shoot induction media. The ideal media combination is MS supplemented with BAP (0.25 mgl⁻¹), Kinetin (0.5 mgl⁻¹), IAA (0.1 mgl⁻¹) and Ch (100.0 mgl⁻¹) and maintained in growth chamber with following conditions, namely, temperature 25±2°C, relative humidity (RH) of 50-60% and 16h photoperiod with fluorescent light (2000µ mol M⁻²S⁻²) followed by an 8h dark period. After incubation for 3-4 weeks several green shoots were obtained from the callus cultures.

The multiple shoots were transferred on the same medium for elongation. The shoots of length 1.5 cm were than transferred to root induction medium for obtaining roots. After 3-4 weeks well developed roots were formed at the base of shoots. The ideal media combination for rooting is MS media supplemented with IBA 2.0 mgl⁻¹. The in vitro rooted shoots resulted in plantlets which were transferred to soil for hardening. The hardened regenerated plantlets were successfully established in soil. The regenerated plants were allowed to grow till maturity.

### In vitro Induction of Shikonin Production In Calli of Arnebia hispidissima

Since callus cultures failed to produce any pigment even after several sub-cultures in the MS media combinations as shown in Table 4, other plant growth regulator combination were studied. Pigmentation with accumulation of Shikonin production was observed only when callus cultures were transferred to culture medium containing 1.0 mgl⁻¹ of IAA instead of 2,4-D and allowed to grow in the dark. Undifferentiated callus tissues of Amebia hispidissima were capable of synthesizing Shikonin derivative or pigment which are normally formed in the cork cell of the roots. Thus, by transferring callus cultures to MS media containing IAA (1.0 mgl⁻¹) and incubating in the dark for 4-5 weeks it was possible to obtain induction of red pigment in the calli. Thus, it was observed that biosynthesis of Shikonin pigment in callus cultures is inhibited by strong auxin and light during tissue culture conditions. Pigment content increased linearly with time after a lag phase of about 4 weeks when callus tissues were grown on culture medium containing IAA in the dark. The callus cultures showing Shikonin induction were transferred to MS media supplemented with 2, 4-D and incubated in the dark for several weeks. It was found that pigment content decreased with time. The interesting observation is that pigment content decreased significantly when 2,4-D was substituted for IAA even through the callus cultures were incubated in the dark.

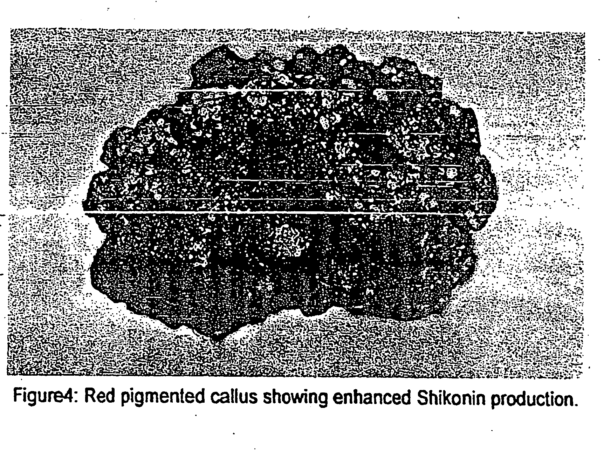

Several experiments were carried out for in vitro induction of Shikonin pigment production in callus cultures. Various explants were cultured on different media combination for production of callus and induction of Shikonin as shown in Table 5. The ideal media combination was MS supplemented with BAP (0.25 mgl⁻¹) and IAA (1.0 mgl⁻¹) for production of callus as well as induction of Shikonin production (Figure 4). It was evident that Shikonin was produced only if the callus cultures were incubated under dark conditions.

Table 5 also shows the ideal media combination for callus production with Shikonin induction. Callus was produced using MS media containing Kinetin in the range of 0.25 - 1.0 mgl⁻¹ and 2,4-D in the range of 0.5-2.0 mgl⁻¹. Callus was also produced using MS media containing BAP in the range of 0.25-1.0 mgl⁻¹ and 2,4-D in the range of 0.5-2.0 mgl⁻¹. This combination did not produce any Shikonin pigment even under dark incubation conditions of 12-24h. The callus culture was subcultured on MS medium containing Kinetin in the range of 0.2-1.0 mgl⁻¹ and IAA in the range of 0.25-2.0 mgl⁻¹ which induced the production of Shikonin pigment. Higher production of Shikonin pigment was observed when callus was cultured on MS media containing BAP in the range of 4.1-1.5 mgl⁻¹ under dark period of 12-2h. Thus, the ideal media combination for Shikonin production in callus cultures is MS medium containing BAP (0.25 mgl⁻¹), IAA (1-0 mgl⁻¹) and CH(100.0 mgl⁻¹). This media combination was found to be ideal for callus production as well.

The effect of auxins was tested for the production of Shikonin in calli of Arnebia hispidissima. The calli which were produced using 2,4-D (1.0 mgl⁻¹) and BAP (0.25-1.0 mgl⁻¹) and subsequently cultured for several weeks in the same medium in the presence of light were transferred to MS media containing IAA (1.0 mgl⁻¹) and BAP(0.25-1.0 mgl⁻¹) and incubated in the dark for 4-5 weeks for Shikonin production. These calli produced Shikonin clearly demonstrating that the potential for pigment synthesis is not lost during the long term culturing in the presence of 2,4-D.

Another observation was that pigment synthesis is repressed by irradiating the callus cultures in white light. For example, old callus cultures grown in MS media containing IAA and Kinetin and under illumination produced no pigments during 4-6 weeks of culturing but pigments were produced under continuous dark period at 25°C. Comparative studies of Cytokinin and Auxins showed that Kinetin is not necessary for Shikonin production. It was seen that Shikonin pigment was present in MS media combination with growth regulators: Kinetin, IAA and BAP IAA but more percent in BAP, IAA combination (80.0%). The total Shikonin content in callus was measured as 0.50 mgg⁻¹ fresh weight of the tissue at the end of the 50 days of the culture period (Table 9).

### Agrobacterium rhizogenes-mediated genetics transformation of Amebia hispidissima

Plant of Arnebia hispidissima were employed for establishing Hairy Root Cultures of Arnebia hispidissima. Various explants namely, leaf, shoot tip, nodal and internodal segments were employed for genetic transformation studies with Agrobacterium rhizogenes wild type strain A4. The leaf explant showed best response (70.7%) as compared to other explant : shoot tip (52%) nodal segment (38.7%) and internodal segment (9.3%) as shown in Table 8. Genetic transformation of leaf explant derived from in vitro grown plants of Amebia was achieved with Agrobacterium rhizogenes wild type strain A4 Source ATCC. The Hairy Root Cultures when inoculated on MS semisolid medium did not produce Shikonin and its derivatives. However, when Hairy Root Cultures inoculated in semisolid or liquid root culture media (RC) as shown in Table 7, the roots produced a large amount of Shikonin, which was released into the medium and quantified on basis of OD₆₀₀. It was observed that Shikonin production by Hairy Roots also showed light dependent inhibition of Shikonin biosynthesis similar to that of Amebia hispidissima cell cultures. The work demonstrates the effectiveness of coculture for the production of plant secondary metabolites. This is the first report of induction of Shikonin production in Hairy Root Cultures of an important medicinal plant Arnebia hispidissima by employing Agrobacterium rhizogenes-mediated genetic transformation.

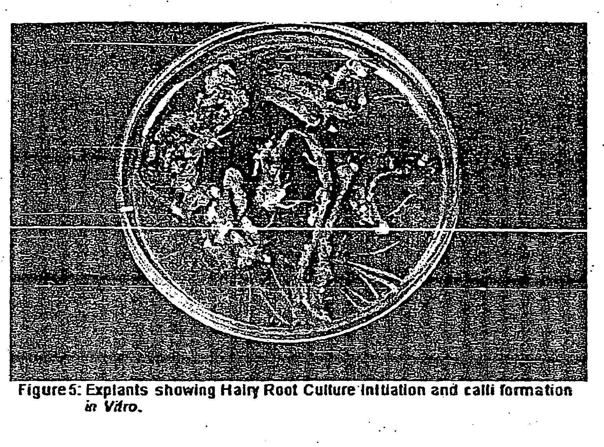

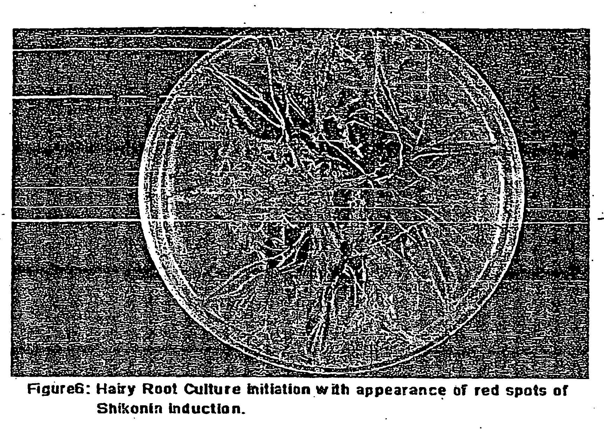

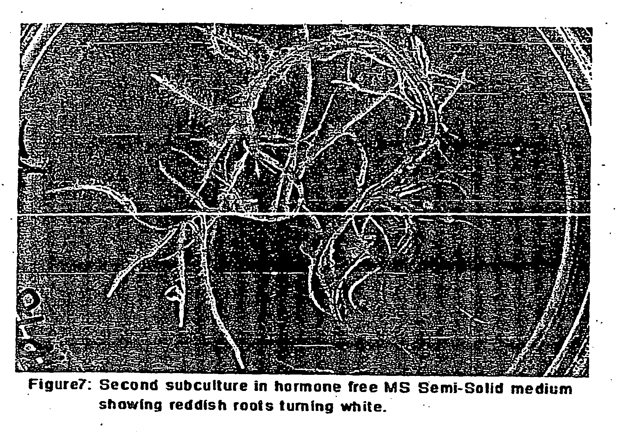

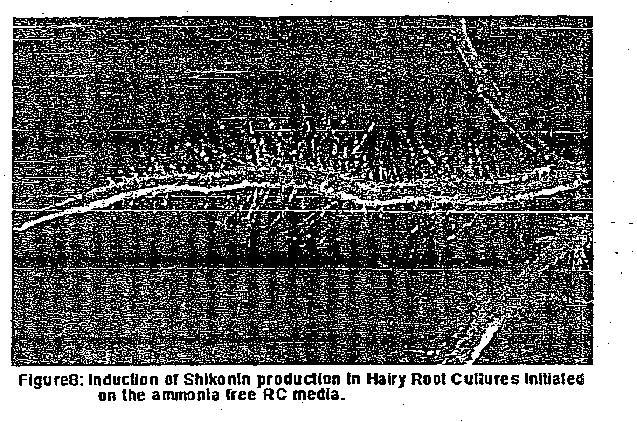

Plants of Arnebia hispidissima were maintained in vitro as multiple shoots clusters. Shoots tip, nodal, internodal and leaf explants were pre cultured for three weeks at low light intensity on MS medium with acetosyringone 100 micro molar. The etiolated explants grown under low light intensity were infected with A.rhizogenes strain A4 wild type and kept at low light in Petri plates. Five days after the co-cultivation in MS medium with acetosyringone 100 micro molar, explants became thick, shriveled up and expanded 1-2 times, were transferred onto agar plates supplemented with antibiotic medium 250 mgl-1 Cefotaxime. Within two weeks Hairy Roots begin to appear at the cut surfaces and Hairy Root Cultures also formed calli on this medium (Figure 5). After the elimination of Agrobacterium on Cefotaxime supplemented media, the cultures were transferred to hormone free MS solid medium, where the formation of Hairy Roots with red spots of Shikonin induction inn the older parts of the tissue are also observed (Figure 6). However, the reddish roots turned white after a second subculture to the same medium (Figure 7), Therefore, it was concluded that MS medium was not suitable for the production of Shikonin in Hairy Root Cultures. The high ammonium content of the MS medium may be responsible for inhibiting Shikonin production (Fujita et. Al. 1981 a, b). Hairy Roots Cultured on the ammonium free RC medium showed fast growth and produced large amount of Shikonin derivatives which were released into the medium (Figure 8). After 3-4 weeks of culture in the RC medium, Shikonin production by Hairy Root Cultures increased as compared to the normai roots. However, it has been observed that addition of charcoal in the RC medium inhibited Shikonin production. The data obtained in the study shows that initiation of Hairy Root Cultured is a useful system for production of Shikonin and its derivatives and leaf explants shows the best response (70.7%) as compared to other explants namely : Shoot tip (52%), nodal segment (38.7%) and intemodal segment (9.3%). The total Shikonin content was found to be 0.85mgg⁻¹ of Hairy roots at the end of 50 days of culture (Table 10).

This invention will be explained in grater details with the help of the accompanying examples :

### Example: 1 High frequency Direct regeneration of plantlets from Shoot tip cultures:

a) Selection and preparation of explants :
   Field grown plants were obtained from Guru Jambheshwar University of Science and Technology, Hisar, Haryana, India.
   For shoot tip cultures, shoot tips (4-6 mm long) which consist of 10-15 developing leaves surrounding the meristem were selected. Bud scales and outer developing leaves were removed and the shoot tip was excised using a sharp, sterile scalpel blade. All the explants washed with mild detergent (Teepol) using standard procedures followed by several rinses in tap water. These were then surface sterilized with 0.1% mercuric chloride (HgCl₂) for 5-7 minutes and subsequently, washed three times with double distilled water to remove traces of mercuric chloride. Explants were placed with the abaxial side down on medium in glass jars with caps.
b) Media preparation and culture conditions : Tissue culture media was prepared by employing the standard MS media (Murashige and Skoog, 1962) containing sucrose (3%) and bacteriological agar (0.8%). Growth regulators were incorporated into the media and the pH was adjusted to 5.8. The media was then sterilized in a autoclave at 15 psi at 121°C for 20 minutes. The growth chamber conditions maintained for in vitro cultures were 25±2°C, 50-60% relative humidity (RH) with 16h photoperiod with fluorescent light (1000 mol M⁻² S⁻¹ photosensitivity),followed by an 8h dark period. Unless or otherwise stated, all growth regulators added to MS medium are in micro molar concentration. The MS media constituents are given in Table 1.
c) Initiation and proliferation of shoot regeneration :
   Shoot tips were cultured on various media combinations such as MS medium alone or supplemented with one of the following plant growth regulators; 6-Benzyl amino purine (BAP, 0.25, 0.5, 1.0 mgl⁻¹); Kinetin (KIN, 0.25, 0.5, 1.0 mgl⁻¹); Indole-3-aceticacid (IAA, 0.1, 0.25, 0.5, 1.0 mgl⁻¹) and casein hydrolysate 100.0 mgl⁻¹ (Table 2). This study showed that MS media supplemented with BAP (0.25 mgl⁻¹); Kinetin (0.5 mgl⁻¹); IAA(0.1 mgl⁻¹) and Casein hydrolysate (100.0 mgl⁻¹) was an ideal media combination for shoot regeneration. This combination led to a high percentage of shoot regeneration from shoot tips (Table 2). It was observed that 1.5-5 cm long linear and lanceolate leaves developed within 4-7 weeks in 93.3% of cultures resulting into a cluster formation at the end of 3-4 weeks of cultures.
   MS media containing only BAP hormone was ineffective in inducing shoot proliferation. Generally, shoot tip explants are more responsive than nodal explants for shoot regeneration. On media supplemented with various BAP, Kinetin and IAA concentrations, 93.3% of shoot tip explants and 60% nodal explants developed more than 10 shoots (Table 2). Tissue culture response, viability, intensity of shoot development and quality of shoots (as measured by the case of dividing shoot cultures, shoot elongation and manifestation of abnormalities such as fasciation, vitrification or etiolation) was recorded weekly for upto 8 weeks. The numbers of shoots regenerated from various explants were analyzed after 4 weeks. This experiment was repeated several times. Flowering was also induced in some of the cultures in vitro. The calyx of the in vitro induced flowers was 6-8 mm long divided almost to base, very hispid; and the corolla was yellow densely pubescent outside at base and apex. Stamens were inserted at or in the throat and half exerted in the short style flowers; stigma small flattened on inside, rounded outside. The induced flowering appeared phenotypically same as in the field grown plants.
d) induction of rooting in regenerated shoots
   Shoots longer than 15 mm length with four or more leaves were excised out and transferred to various media combinations such as, MS medium alone or in combination with supplemented adjuvants with one of the following combinations of growth regulators: Indole-3-butyeric acid (IBA, 0.5 1.0, 1.5, 2.0 mgl⁻¹), α-naphthalene acetic acid (NAA, 0.5, 1.0 2.0 mgl⁻¹), indole-3-acetic acid (IAA, 1-0, 2.0, 2.5, 3.0 mgl⁻¹) with or without Charcoal (0.1%) or half strength MS media alone or half strength MS media supplemented with one of the following combination of growth regulators: IBA (0.5, 1.0, 2.0 mgl⁻¹), NAA (0.5, 1.0, 2.0 mgl⁻¹), IAA (1.0, 2.1, 2.5, 3.0 mgl⁻¹)
   The number of rooted shoots and quality of roots was recorded after three weeks of culture. Among the various auxins investigated, IBA (2.0 mgl⁻¹) was most effective in initiating roots on shoots isolated from shoots induced in culture media (Table 3). Shlkonln production was observed in the roots. There was higher amount of Shikonin production in older roots as compared to younger ones.
   Hormones, namely, IAA and NAA were less effective for root induction. It was also observed that addition of charcoal (0.1%) in both MS medium and half strength MS medium with IBA (2.0 mgl⁻¹) inhibited the Shikonin pigment production. The ideal media combination for root induction is MS media supplemented with Kinetin (0.5 mgl⁻¹), IAA (0.1 mgl⁻¹), BAP (0.25 mgl⁻¹), and CH (100.0 mgl⁻¹).
e) Acclimatization of regenerated plantlet and field trials
   Rooted explants with four or more leaves and three to four roots were washed to remove the adhering sucrose/agar medium and then transferred to pots containing a mixture of sand : vermiculite compost in the ratio 2:1 (v/v) and exposed to natural light and 70±5% Relative humidity in the green house. The light weight and porous texture of soil substitute prevents root damage, allows better root growth and hence development of a healthier plant. The whole process took 2-3 months from the start until a regenerated plant could be transferred to the green house and field plantations. The tissue culture plants appeared phenotypically normal and resembled the wild type plants growing in field.

### Example 2 : High frequency direct regeneration of plants from nodal segments :

Nodal segments were prepared by cutting the stem into 5-10 mm length, where as each node segment have an axillary bud. Nodal segments were from third to sixth node from the tip of the branch.

These explants served as the basic material for the present invention.

The nodal segment explants were surface sterilized as described in Example 1. Tissue culture media was prepared by employing the standard MS media (Murashige and Skoog, 1962) containing sucrose (3%) and bacteriological agar (0.8%), as described in Example 1 (basal media) Growth regulators were incorporated into the media and the pH was adjusted to 5.8. The details of media preparation and sterilization are as described in Example 1. The treated explants were cultured on MS medium alone or supplemented with one of the following growth regulators; 6-Benzylaminopurine (BAP; 0.25, 0.5, 1.0 mgl⁻¹); Kinetin (KIN; 0.25, 0.5, 1.0 mgl⁻¹); lndate-3-aceticacid (IAA, 0.1, 0.25, 0.5, 1.0 mgl⁻¹). MS media supplemented with BAP (0.25 mgl⁻¹), Kinetin (0.5 mgl⁻¹), IAA (0.1 mgl⁻¹) and CH (100.0 mgl⁻¹) showed 1.5-2.5 cm long leaves developed within 4-6 weeks. So considerable shoot elongation was observed in the presence of BAP (0.25 mgl⁻¹), Kinetin (0.5 mgl⁻¹) and IAA (0.1 mgl⁻¹) Shoot tip cultured on MS medium supplemented with BAP (0.25 mgl⁻¹), Kinetin (0.5 mgl⁻¹), IAA (0.1 mgl⁻¹) and Casein hpdrolysate (100.0 mgl⁻¹) produced more than 60 percent shoot regeneration from nodal segments (as shown in Table 2). Thus the ideal combination of BAP, Kinetin and IAA is identified for effective and enhanced shoot regeneration from the various explants.

Further, shoots were allowed to elongate in the same medium up to a length of 1.5 to 2 centimeters and were cultured on root induction medium for rooting. Different rooting media combinations were tested for root induction and these combinations are shown in Table 3. The ideal media combination for root inductions is MS medium supplemented with Indole-3-butyric acid (IBA 2.0 mgl-1). The in vitro rooted shoots resumed growth after a short period of acclimatization and resulted in plantlets which were transferred to the soil after hardening. These regenerated plantlets were successfully established in soil. In vitro flowering of tissue culture raised plantlets were observed when these plantlets were maintained for long periods in tissue culture media.

For root induction shoots longer than 1.5 cm, with four or more leaves were excised out and transferred to MS medium alone or in combination with supplemented adjuvants with one of the following combinations of growth regulators: Indole-3-butyeric acid (IBA, 0.5, 1.0, 1.5, 2.0 mgl⁻¹), α-naphthalene acetic acid (NAA, 0.5, 1.0, 2.0 mgl⁻¹), indole-3-acetic acid (IAA, 1.0, 2.0, 2.5, 3.0 mgl⁻¹) with and without Charcoal (0.1%) or half strength MS alone or half strength MS supplemented with one of the following combination of growth regulators: IBA (0.5, 1.0, 2.0 mgl⁻¹), NAA (0.5, 1.0, 2.0 mgl⁻¹), IAA (1.0, 2.0, 2.5, 3.0 mgl⁻¹). Among the various auxins investigated, IBA (2.0 mgl⁻¹) was most effective in initiating roots on shoots isolated from shoots induced in culture media (Table 3).

### Rooted plantlets were then transferred to pots containing a mixture of sand :

Vermiculite compost in the ratio 2:1 (v/v) and exposed to natural light and 70±5% Relative humidity in the green house for acclimatization and hardening of the regenerated plantlets, the details are as given in Example 1.

### Example 3: Initiation of callus and induction of Shikonin production from Arnebia hispidissima:

Explants were selected from shoot tips, leaf segments, nodal segments and internodal segments. Nodal segments were prepared by cutting the stem into 5-10 mm length, where as each segment have an axillary bud. Nodal segments were from third to sixth node from the tip of the branch. The nodal segment explants were surface sterilized as described in Example 1. The surface sterilized explants were cultured on various media for callus induction. The various media combinations are given in Table 1. Tissue culture media was prepared by employing the standard MS media (Murashige and Skoog, 1962) containing sucrose (3%) and bacteriological agar (0.8%) as described in Example 1 (basal media) with various plant growth regulators combination.

Callus induction from various explants of Arnebia hispidissima using various media supplemented is shown in Table 4 [MS + Kinetin (0.2-2.0) + 2,4-D (0.5-2.0) + CH (50-150) mgl⁻¹]. The ideal media for callus induction was observed to be MS medium supplemented with Kinetin (0.5 mgl⁻¹), 2,4-D(1.0 mgl⁻¹) and CH (100.0 mgl⁻¹) under photoperiod of 12h at 25±2°C showed 86.7±0.09 percent callus induction from nodal explants (Table 4).

After producing callus on MS media combinations with Kinetin (0.2-2.0 mgl⁻¹), 2,4-D (0.5-2.0 mgl⁻¹) and CH (50-150 mgl⁻¹) under 6-12h photoperiods at 25±2°C, the callus was sub cultured after 4 weeks on media containing MS medium, Kinetin (0.2-2.0 mgl⁻¹), IAA (0.01-2.0 mgl⁻¹) and CH (50-150 mgl⁻¹). Callus started producing Shikonin pigment under photoperiod of 6-12h per day at 25±2°C but more pigmented callus was observed under dark conditions of 12-24h at 25±2°C. It was observed that Shikonin derivative content increased when Kinetin was substituted with BAP in the same or less concentration. The ideal conditions for induction of Shikonin pigment was observed on media containing MS medium, BAP 0.25 mgl⁻¹, IAA 1.0 mgl⁻¹ and CH 100.0 mgl⁻¹ as shown in Table 5 under dark condition for 12-24h at 25±2°C.

### Example 4: Preparation of plant material for co-cultivation:

The explants namely; leaf, node, inter node and shoot tip were excised from field grown plant and were surface sterilized with 0.1% mercuric chloride (HgCl₂) for 5-7 minutes and subsequently washed three times with double distilled water to remove traces of HgCl₂ and inoculated on MS media (Table 1), supplemented with BAP 0.25 mgl⁻¹, Kinetin 0.5 mgl⁻¹, IAA 0.1 mgl⁻¹, CH 100.0 mgl⁻¹ and shoots obtained were multiplied on the same medium for 3 weeks at low light intensity. This led to the formation of clusters of multiple shoots. The leaf, shoot tip, nodal and internodal explants were obtained from in vitro grown plants served as starting material for co-cultivation and genetic transformation of Arnebia hispidissima.

### Example 5: Preparation of LB medium for Agrobacterium rhizogenes-mediated culture initiation.

All the components of Luria Broth LB) media (Table 6) were dissolved in distilled water and final volume was made up to one litre. The pH of the medium was adjusted to 7.0 with NaOH solution and agar was added to the above medium. After melting the agar in microwave, measured quantity (50 or 100 ml) of medium was dispensed in each flask or test tube and autoclaved at 15 psi at 121°C for 15-20 minutes.

### Example 6: Agrobacterium culture initiation

From the 25% glycerol stocks v/v of Agrobacterium rhizogenes wild type strain A4 culture was streaked on to LB Petri-plate by using the inoculation loop and incubated at 28°C for 12-24h. Single colonies of Agrobacterium rhizogenes were further streaked for three successive days on solid LB medium (Table 6) for 24h and grown at 28°C. The single colony from the actively growing culture was inoculated in 30 ml liquid LB medium (without agar) at 28°C at 200 rpm in incubator shaker. Bacterial cultures were allowed to grow to an absorbance of 0.8-1.0 OD₆₀₀. From the actively growing cultures, fresh inoculation was made in LB and allowed to grow under similar conditions mentioned above till the OD_{600.} of 0.30 was obtained.

### Example 7 : Infection of Arnebia explants with Agrobacterium rhizogenes

The Agrobacterium cells were pelleted at 3000 rpm for 10 min. The supernatant was discarded leaving only the pellet which was immediately re-suspended in 30 ml liquid MS(without vitamins, sucrose and hormones) with 100µM acetosyringone (Aldrich Sigma Chemicals Co; USA). The Agrobacterium rhizogenes cell suspension so obtained in, the MS media without vitamin, hormones and sucrose was poured in the sterilized plate and in vitro produced explants were placed in the Petri plates. The plate was sealed with Para film M and stirred (50 rpm) for 15-20 minutes to ensure that the cut surface got wet. Explants were blotted dry on sterilized filter paper, to get rid off the excess bacteria adhering to the tissue.

### Example 8: Co-cultivation of Explants:

The infected explants were co-cuitivated in Petri dishes containing semi-solid MS medium supplemented with or without IBA 2.0 mgl⁻¹ and 100µM acetosyringone. Petri plates were sealed with Para film M.. These cultures were incubated for 5 days in complete dark for 24h at 25±2°C.

### Example 9: Selection of transformed tissue:

Five days after the inoculation, shoots were transferred onto same medium as above and supplemented with antibiotic 250 mgl⁻¹ Cefotaxime. Two weeks after the inoculation, the Hairy Roots appeared at the infection sites. They were separated by using scalpel blades and sub-cultured on MS semi-solid medium "hormone free" as well as root culture liquid medium (RC), as shown in Table 7. The cultures were kept on a rotary shaker at 100 rpm, at 25°C in the dark for 24 h.

### Example 10 : Extraction of Shikonin derivatives:

The protocol for extraction of Shikonin content and its derivatives in Hairy Roots were optimized using the method proposed by Mizukami et.al. 1997, Yazaki et. Al. 1998. Fukui et. Al 1998). Hairy Roots were homogenized with Chloroform (CHCl₃) and left in the dark for a range of 12-24h. After drying over MgSO₄ the extract was evaporated to dryness and 2.5% KOH solution was added. Blue colour developed after 10 min.The Shikonin content was assayed by employing UV Spectrophotometer and absorbance was measured at 622 nm as described by Fukui et. Al (1998).

| **Table 1. Composition of MS (Murashige and Skoog, 1962) Medium** | | | | | |
|---|---|---|---|---|---|
| **Sr. No.** | **Strength** | **Constituent salts** | **Quantity** | **Use ml/l** | **Actual amount in culture medium, mgl⁻¹** |
| 1 | X 20 | | 2 Litre. | 50 | |
| | | NH₄NO₃ | 66.0 g | | 1650 |
| | | KNO₃ | 76.0 g | | 1950 |
| | | KH₂PO₄ | 6.8 g | | 170 |
| | | H₃BO₃ | 0.248 g | | 6.2 |
| | | MnSO₄.7H₂O | 0.892 g | | 22.3 |
| | | ZnSO4.7H O | 0.344 g | | 8.6 |
| | | KI | 0.033 g | | 0.825 |
| | | (a)*CuSO₄ 5H₂O | 1.0 ml | | 0.025 |
| | | (b)Na₂MoO₄2H₂O | 1.0 ml | | 0.25 |
| | | (c) CoCl₂.2H₂O | 1.0 ml | | 0.025 |
| | | | ½ Litre | | |
| 2 | X 50 | CaCl₂.2H₂O | 11.0 g | 20 | 440 |
| 3 | X 50 | MgSO₄.7H₂O | 9.250 g | 20 | 370 |
| | | | 1 Litre | | |
| 4 | X 100 | FeSO₄.7H₂O | 2.780 g | 10 | 27.8 |
| | | Na₂EDTA | 3.728 g | | 37.28 |
| | | | 1 Litre | | |
| 5 | X 100 | Thiamine HCl | 0.010 g | 10 | 0.1 |
| | | Nicotinic acid | 0.050 g | | 0.5 |
| | | Pyridoxine HCl | 0.050 g | | 0.5 |
| | | Glycine | 0.20 g | | 2 |
| | | **Myoinositol | 100 mgl⁻¹ | | 100 |
| | | Sucrose | 30 gl⁻¹ | | 30,000 |

| | | | | | |
|---|---|---|---|---|---|
| (a)* Dissolved 100 mg (CuSO₄.5H₂O) in 100 ml distilled water. (b) Dissolved 1 g (Na₂MoO₄.2H₂O) in 100 ml distilled water. (c) Dissolved 100 mg (CoCl₂.2H₂O) in 100 ml distilled water. NOTE: -- 1. Add one ml of Conc. HCl in stock solution No. 1 to avoid precipitation. 2. Slightly Heat stock solution No. 4 to avoid precipitation. **:-- Myoinositol and sucrose are added in solid form while preparing the final volume. pH 5.8 | | | | | |

| **Table 2. Direct Plant Regeneration from different explants of *Arnebia hispidissima*** | | | |
|---|---|---|---|
| **Treatment (mg L⁻¹)** | **Explant (number of replicate)** | **Number of shoot regenerated** | **Percentage of shoot regeneration per 15 explants** |
| MS (Control) | Shoot tip (15) | 0 | 0.0 ± 0.00 |
| | Nodal segment 15) | 0 | 0.0 ± 0.00 |
| | Inter nodal (15) | 0 | 0.0 ± 0.00 |
| | Leaf segment (15) | 0 | 0.0 ± 0.00 |
| MS+IAA0.1+Kin 0.25+BAP 0.25 | Shoot tip (15) | 8 | 53.3 ± 0.13 |
| | Nodal segment(15) | 6 | 40.0 ± 0.13 |
| | Inter nodal (15) | 0 | 0.0 ± 0.00 |
| | Leaf segment (15) | 2 | 13.3 ± 0.09 |
| MS+IAA0.1+Kin 0.25+BAP 0.5 | Shoot tip (15) | 8 | 53.3 ± 0.13 |
| | Nodal segment(15) | 7 | 46.7 ± 0.13 |
| | Inter nodal (15) | 0 | 0.0 ± 0.00 |
| | Leaf segment (15) | 0 | 0.0 ± 0.00 |
| MS+IAA0.1+Kin 0.25+BAP 1.0 | Shoot tip (15) | 5 | 33.3 ± 0.13 |
| | Nodal segment 15) | 6 | 40.0 ± 0.13 |
| | Inter nodal (15) | 0 | 0.0 ± 0.00 |
| | Leaf segment (15) | 1 | 06.7 ± 0.07 |
| MS+IAA0.1+Kin 0.5+ BAP 0.25 | Shoot tip (15) | 14 | 93.3 ± 0.00 |
| | Nodal segment (15) | 9 | 60.0 ± 0.13 |
| | Inter nodal (15) | 0 | 0.0 ± 0.00 |
| | Leaf segment (15) | 3 | 20.0 ± 0.11 |
| MS+IAA0.1+Kin 0.5+BAP 0.5 | Shoot tip (15) | 7 | 46.7 ± 0.13 |
| | Nodal segment 15) | 8 | 53.3 ± 0.13 |
| | Inter nodal (15) | 0 | 0.0 ± 0.00 |
| | Leaf segment (15) | 0 | 0.0 ± 0.00 |
| MS+IAA0.1+Kin 0.5+BAP 1.0 | Shoot tip (15) | 7 | 46.7 ± 0.13 |
| | Nodal segment(15) | 4 | 26.7 ± 0.12 |
| | Inter nodal (15) | 0 | 0.0 ± 0.00 |
| | Leaf segment (15) | 1 | 06.7 ± 0.07 |
| MS+IAA0.1+Kin 1.0+BAP 0.25 | Shoot tip (15) | 10 | 66.7 ± 0.13 |
| | Nodal segment 15) | 7 | 46.7 ± 0.13 |
| | Inter nodal (15)) | 0 | 0.0 ± 0.00 |
| | Leaf segment (15) | 0 | 0.0 t 0.00 |
| MS+IAA0.1+Kin 1.0+BAP 0.5 | Shoot tip (15) | 10 | 66.7 ± 0.13 |
| | Nodal segment 15) | 6 | 40.0 ± 0.13 |
| | Inter nodal (15) | 0 | 0.0 ± 0.00 |
| | Leaf segment (15) | 1 | 06.7 ± 0.07 |
| MS+IAA0.1+Kin 1.0+BAP 1.0 | Shoot tip (15) | 7 | 46.7 ± 0.13 |
| | Nodal segment 15) | 5 | 33.3 ± 0.13 |
| | Inter nodal (15) | 0 | 0.0 ± 0.00 |
| | Leaf segment (15) | 0 | 0.0 ± 0.00 |

| **Table 3. Root induction in shoots regenerated from *Arnebia hispidissima* on MS Media supplemented with or without IBA after 3 weeks of culture** | | | |
|---|---|---|---|
| **Treatment (mgL⁻¹)** | **Total number of replicate** | **Number of cultures showing rooting** | **Rooting percentage** |
| MS (control) | 15 | 0 | 0 |
| ½ MS (control) | 15 | 0 | 0 |
| ½ MS + IBA 0.5 | 15 | 0 | 0 |
| MS + iBA 0.5 | 15 | 5 | 33 |
| ¹/₂ MS + IBA 1.0 | 15 | 2 | 13 |
| MS + IBA 1.0 | 15 | 7 | 47 |
| ¹/₂ MS + IBA 1.5 | 15 | 6 | 40 |
| MS + IBA 1.5 | 15 | 8 | 53 |
| ¹/₂ MS + IBA 2.0 | 15 | 8 | 53 |
| **MS + IBA 2.0** | **15** | **12** | **80** |
| MS + IBA 2.0 + IAA 0.1 | 15 | 6.5 | 43 |
| MS + IBA 2.0 + Charcoal 0.1 | 15 | 9.5 | 63 |

| | | | |
|---|---|---|---|
| IBA: Indole Butyric Acid IAA: Indole 3-Acetic Acid | | | |

| **Table 4. Callus induction in explants of *Arnebia hispidissima* in various media after 2-3 weeks. (MS basal + CH 100mgl⁻¹)** | | | | | |
|---|---|---|---|---|---|
| **Treatment (mg**l**⁻¹)** | **Explants (number of replicate)** | **Average Number of Explants formed callus** | **Percentage of Callus Induction per 15 explants +S.E** | **Induction of Shikonin and its derivatives (under fluorescent light)** | **Induction of Shikonin and its derivatives (under dark condition)** |
| MS (control) | Internodal segment 15 | 0 | 0 | Not Seen | Not Seen |
| | Leaf segment 15 | 0 | 0 | | |
| | Nodal segment 15 | 0 | 0 | | |
| | Shoot tip 15 | 0 | 0 | | |
| MS + 2,4-D 0.25 | Internodal segment 15 | 4 | 26.7 ± 0.12 | Green callus but no pigmentation | Yellowish White |
| | Leaf segment 15 | 4 | 26.7 ± 0.12 | | |
| | Nodal segment 15 | 6 | 40.0 ± 0.13 | | |
| | Shoot tip 15 | 5 | 33.3 ± 0.13 | | |
| MS + 2,4-D 0.5 | Internodal segment 15 | 5 | 33.3 ± 0.13 | Green callus pigmentation | Yellowish White |
| | Leaf segment 15 | 3 | 20.0 ± 0.11 | | |
| | Nodal segment 15 | 10 | 66.7 ± 0.13 | | |
| | Shoot tip 15 | 9 | 60.0 ± 0.13 | | |
| MS + 2,4-D 1.0 | Internodal segment 15 | 8 | 53.3 ± 0.13 | Green callus but no pigmentation | Yellowish White |
| | Leaf segment 15 | 6 | 40.0 ± 0.13 | | |
| | Nodal segment 15 | 12 | 80.0 ± 0.11 | | |
| | Shoot tip 15 | 9 | 60.0 ± 0.13 | | |
| MS + 2,4-D 1.5 | Internodal segment 15 | 5 | 33.3 ± 0.13 | Green callus but no pigmentation | Yellowish White |
| | Leaf segment 15 | 5 | 33.3 ± 0.13 | | |
| | Nodal segment 15 | 9 | 60.0 ± 0.13 | | |
| | Shoot tip 15 | 8 | 53.3 ± 0.13 | | |
| MS + 2,4-D 2.0 | Internodal segment 15 | 4 | 26.7 ± 0.12 | Green callus but no pigmentation | Yellowish White |
| | Leaf segment 15 | 6 | 40.0 ± 0.13 | | |
| | Nodal segment 15 | 8 | 53.3 ± 0.13 | | |
| | Shoot tip 15 | 7 | 46.7 ± 0.13 | | |
| MS + Kin 0.25 + 2,4-D 1.0 | Internodal segment 15 | 7 | 46.7 ± 0.13 | Green callus but no pigmentation | Yellowish White |
| | Leaf segment 15 | 6 | 40.0 ± 0.13 | | |
| | Nodal segment 15 | 9 | 60.0 ± 0.13 | | |
| | Shoot tip 15 | 8 | 53.3 ± 0.13 | | |
| MS + Kin 0.5 + 2,4-D 1.0 | Internodal segment 15 | 10 | 66.7 + 0.13 | Green callus but no pigmentation | Yellowish White |
| | Leaf segment 15 | 9 | 60.0 + 0.13 | | |
| | Nodal segment 15 | 13 | 86.7 + 0.09 | | |
| | Shoot tip 15 | 9 | 60.0 + 0.13 | | |
| MS + Kin 1.0 + 2.4-D 1.0 | Internodal segment 15 | 9 | 60.0 ± .0.13 | Green callus but no pigmentation | Yellowish White |
| | Leaf segment 15 | 8 | 53.3 ± 0.13 | | |
| | Nodal segment 15 | 11 | 73.3 ± 0.12 | | |
| | Shoot tip 15 | 9 | 60.0 ± 0.13 | | |
| MS + Kin 1.5 + 2,4-D 1.0 | Internodal segment 15 | 5 | 33.3 ± 0.13 | Green callus but no pigmentation | Yellowish White |
| | Leaf segment 15 | 5 | 33.3 ± 0.13 | | |
| | Nodal segment 15 | 8 | 53.3 + 0.13 | | |
| | Shoot tip 15 | 8 | 53.3 ± 0.13 | | |
| MS + Kin 2.0 + 2,4-D 1.0 | Internodal segment 15 | 3 | 20.0 ± 0.11 | Green callus but no pigmentation | Yellowish White |
| | Leaf segment 15 | 2 | 13.3 ± 0.09 | | |
| | Nodal segment 15 | 7 | 46.7 ± 0.13 | | |
| | Shoot tip 15 | 5 | 33.3 ± 0.13 | | |
| MS + BAP 0.25 + 2,4-D 1.0 | Internodal segment 15 | 7 | 46.7 ± 0.13 | Green callus but no pigmentation | Yellowish White |
| | Leaf segment 15 | 6 | 40.0 ± 0.13 | | |
| | Nodal segment 15 | 10 | 66.7 ± 0.13 | | |
| | Shoot tip 15 | 8 | 53.3 ± 0.13 | | |
| MS + BAP 0.5 + 2,4-D 1.0 | Internodal segment 15 | 8 | 53.3 ± 0.13 | Green callus but no pigmentation | Yellowish White |
| | Leaf segment 15 | 8 | 53.3 ± 0.13 | | |
| | Nodal segment 15 | 12 | 80.0 ± 0.11 | | |
| | Shoot tip 15 | 5 | 33.3 ± 0.13 | | |
| MS + BAP 1.0 + 2,4-D 1.0 | Internodal segment 15 | 9 | 60.0 ± 0.13 | Green callus but no pigmentation | Yellowish White |
| | Leaf segment 15 | 8 | 53.3 ± 0.13 | | |
| | Nodal segment 15 | 10 | 66.7 ± 0.13 | | |
| | Shoot tip 15 | 7 | 46.7 ± 0.13 | | |
| MS + BAP 1.5 + 2,4-D 1.0 | Internodal segment 15 | 7 | 46.7 ± 0.13 | Green callus but no pigmentation | Yellowish White |
| | Leaf segment 15 | 5 | 33.3 ± 0.13 | | |
| | Nodal segment 15 | 7 | 46.7 ± 0.13 | | |
| | Shoot tip 15 | 5 | 33.3 ± 0.13 | | |
| MS + BAP 2.0 + 2.4-D 1.0 | Internodal segment 15 | 6 | 40.0 ± 0.13 | Green callus but no pigmentation | Yellowish White |
| | Leaf segment 15 | 3 | 20.0 ± 0.11 | | |
| | Nodal segment 15 | 7 | 46.7 ± 0.13 | | |
| | Shoot tip 15 | 5 | 33.3 ± 0.13 | | |

| **Table 5. Shikonin induction in callus of *Arnebia hispidissima* in various media after 2-3 weeks. (MS basal + CH 100mgl⁻¹)** | | | | | |
|---|---|---|---|---|---|
| **Treatment (mgl⁻¹)** | **Explants (number of replicate)** | **Average Number of Explants formed callus** | **Percentage of Callus Induction per 15 explants +S.E** | **Induction of its derivatives (under fluorescent light)** | **Induction of Shikonin and its derivatives (under dark condition)** |
| MS + IAA 0.25 | Internodal segment 15 | 4 | 26.7 ± 0.12 | Callus with reddish tinge | Pigmentation in few cell aggregates |
| | Leaf segment 15 | 2 | 13.3 ± 0.09 | | |
| | Nodal segment 15 | 5 | 33.3 ± 0.13 | | |
| | Shoot tip 15 | 4 | 26.7 ± 0.12 | | |
| MS + IAA 0.5 | Internodal segment 15 | 3 | 20.0 ± 0.11 | Callus with reddish tinge | Pigmentation in few cell aggregates |
| | Leaf segment 15 | 3 | 20.0 ± 0.11 | | |
| | Nodal segment 15 | 6 | 40.0 ± 0.13 | | |
| | Shoot tip 15 | 4 | 26.7 ± 0.12 | | |
| MS + IAA 1.0 | Internodal segment 15 | 4 | 26.7 ± 0.12 | Callus with reddish tinge | Pigmentation in few cell aggregates |
| | Leaf segment 15 | 5 | 33.3 ± 0.13 | | |
| | Nodal segment 15 segment | 10 | 66.7 ± 0.13 | | |
| | Shoot tip 15 | 9 | 60.0 ± 0.13 | | |
| MS + IAA 1.5 | Internodal segment 15 | 3 | 20.0 ± 0.11 | Callus with reddish tinge | Pigmentation in few cell aggregates |
| | Leaf segment 15 | 4 | 26.7 ± 0.12 | | |
| | Nodai segment 15 segment | 9 | 60.0 ± 0.13 | | |
| | Shoot tip 15 | 8 | 53.3 ± 0.13 | | |
| MS + IAA 2.0 | Internodal segment 15 | 2 | 13.3 ± 0.09 | Callus with reddish tinge | Pigmentation in few cell aggregates |
| | Leaf segment 15 | 4 | 26.7 ± 0.12 | | |
| | Nodal segment 15 | 7 | 46.7 ± 0.13 | | |
| | Shoot tip 15 | 5 | 33.3 ± 0.13 | | |
| MS + Kin 0.25 + IAA 1.0 | Internodal segment 15 | 7 | 46.7 ± 0.13 | Callus with reddish tinge | Pigmentation in few cell aggregates |
| | Leaf segment 15 | 4 | 26.7 ± 0.12 | | |
| | Nodal segment 15 | 9 | 60.0 ± 0.13 60.0 ± 0.13 | | |
| | Shoot tip 15 | 6 | 40.0 ± 0.13 | | |
| MS + Kin 0.5 +IAA 1.0 | Internodal segment 15 | 10 | 66.7 ± 0.13 | Callus with reddish tinge | Pigmentation in few cell aggregates |
| | Leaf segment 15 | 9 | 60.0 ± 0.13 | | |
| | Nodal segment 15 Nodal segment 15 | 11 | 73.3 ± 0.12 73.3 ± 0.12 | | |
| | Shoot tip 15 | 5 | 33.3 ± 0.13 | | |
| MS + Kin 1.0 + IAA 1.0 | Intemodal segment 15 | 9 | 60.0 ± 0.13 | Callus with reddish tinge | Pigmentation in few cell aggregates |
| | Leaf segment 15 | 7 | 46.7 ± 0.13 | | |
| | Nodal segment 15 | 9 | 60.0 ± 0.13 | | |
| | Shoot tip 15 | 4 | 26.7 ± 0.12 | | |
| MS + BAP 0.25 + IAA 1.0 | Internodal segment 15 | 11 | 73.3 ± 0.12 | Red Pigmented Callus | Best red pigmented Callus and enhanced Shikonin productivity |
| | Leaf segment 15 Leaf segment 15 | 10 | 66.7 + 0.13 | | |
| | Nodal segment 15 | 12 | 80.0 + 0.11 | | |
| | Shoot tip 15 | 7 | 46.7 + 0.13 | | |
| MS + BAP 0.5 + IAA 1.0 | Internodal segment 15 | 9 | 60.0 ± 0.13 | Pigmentation Present | Enhanced Shikonin content |
| | Leaf segment 15 | 7 | 46.7 ± 0.13 | | |
| | Nodal segment 15 | 11 | 73.3 ± 0.12 | | |
| | Shoot tip 15 | 5 | 33.3 ± 0.13 | | |
| MS + BAP 1.0 + IAA 1.0 | Internodal segment 15 | 7 | 46.7 ± 0.13 | Pigmentation Present | Enhanced Shikonin content |
| | Leaf segment 15 | 4 | 26.7 ± 0.12 | | |
| | Nodal segment 15 | 8 | 53.3 ± 0.13 | | |
| | Shoot tip 15 | 7 | 46.7 ± 0.13 | | |

| **Table 6. Composition of Luria Broth (LB) Medium** | | |
|---|---|---|
| **Sr. No.** | **Constituent salts** | **Actual amount in culture medium, gl⁻¹** |
| 1 | Tryptone | 10 |
| 2 | Yeast extract | 0.5 |
| 3 | Sodium Chloride | 10 |
| 4 | Agar | 14 |
| 5 | pH | 7.0 |

| **Table 7. Composition of RC (EMBO Course, 1982) Medium stock solution** | | |
|---|---|---|
| **Sr. No.** | **Constituent salts** | **Actual amount in culture medium, mgl⁻¹** |
| 1 | Ca(NO₃)₂4H₂O | 288 |
| 2 | KNO₃ | 80 |
| 3 | KCl | 65 |
| 4 | MgSO₄.7H₂O | 370 |
| 5 | Na₂SO₄.10H₂O | 226.7 |
| 6 | NaH₂PO₄.4H2O | 21.5 |
| 7 | H₃BO₃ | 1.5 |
| 8 | ZnSO₄.7H₂O | 2.65 |
| 9 | Kl | 0.75 |
| 10 | CuSO₄.5H₂O | 0.025 |
| 11 | NaMoO₄.2H₂O | 0.25 |
| 12 | MnCl₂.4H₂O | 6 |
| 13 | FeCl₃ | 3.2 |
| 14 | Na EDTA | 7.4 |
| 15 | Thiamine HCl | 0.1 |
| 16 | Nicotinic acid | 0.5 |
| 17 | Pyridoxine HCl | 0.1 |
| 18 | Glycine | 3 |
| 19 | Sucrose | 30,000 |
| | pH | 5.7 |

| **Table 8. *Agrobacterium rhizogenes* strain A4 wild type mediated genetic transformation of different explants** | | | | |
|---|---|---|---|---|
| **Experiment Number** | **Number of explants inoculated with Agrobacterium** | **Number of explants forming hairy roots** | **Percentage of hairy roots induction per hairy roots induction per 15 explants ± S.E** | **Mean Percentage of hairy roots induction per 15 explants** |
| **A** | **Leaf segment** | | | |
| 1 | 15 | 12 | 80.0 ± 0.11 | 70.7 |
| 2 | 15 | 10 | 66.7 ± 0.13 | |
| 3 | 15 | 12 | 80.0 ± 0.11 | |
| 4 | 15 | 11 | 73.3 ± 0.12 | |
| 5 | 15 | 8 | 53.3 ± 0.13 | |

| **B** | **Shoot tip** | | | |
|---|---|---|---|---|
| 1 | 15 | 10 | 66.7 ± 0.13 | 52.0 |
| 2 | 15 | 10 | 66.7 ± 0.13 | |
| 3 | 15 | 7 | 46.7 ± 0.13 | |
| 4 | 15 | 8 | 53.3 ± 0.13 | |
| 5 | 15 | 4 | 26.7 ± 0.12 | |

| **C** | **Nodal segment** | | | |
|---|---|---|---|---|
| 1 | 15 | 8 | 53.3 ± 0.13 | 38.7 |
| 2 | 15 | 5 | 33.3 ± 0.13 | |
| 3 | 15 | 8 | 53.3 ± 0.13 | |
| 4 | 15 | 6 | 40.0 ± 0.13 | |
| 5 | 15 | 2 | 13.3 ± 0.09 | |

| **D** | **Inter nodal segment** | | | |
|---|---|---|---|---|
| 1 | 15 | 2 | 13.3 ± 0.09 | 9.3 |
| 2 | 15 | 0 | 00.0 ± 0.00 | |
| 3 | 15 | 4 | 26.7 ± 0.12 | |
| 4 | 15 | 1 | 06.7 ± 0.07 | |
| 5 | 15 | 0 | 00.0 ± 0.00 | |

| **Table 9: Spectrophotometric Analysis of Induction of Shikonin Production in Callus Culture of *Arnebia hispidissima* at Various Subculture Levels** | | | |
|---|---|---|---|
| **Sr. no.** | **Number of Days** | **Optical Density of Shikonin Derivatives*** | **Shikonin (mgg⁻¹)*** |
| 1 | 0 | 0 | 0 |
| 2 | 10 | 0.109 | 0.084 |
| 3 | 20 | 0.265 | 0.164 |
| 4 | 30 | 0.327 | 0.188 |
| 5 | 40 | 0.575 | 0.268 |
| 6 | 50 | 1.010 | 0.50 |

| | | | |
|---|---|---|---|
| *: - Mean of three replicates | | | |

The values refers to the increase in Shikonin Content (mg) of 1g of starting material.

| **Table 10: Spectrophotometric Analysis of Shikonin Induction *In vitro* Hairy Root Cultures of *Arnebia hispidissima* in Hormone Free Root Culture Medium for Varying Periods of Time** | | | |
|---|---|---|---|
| **Sr. No.** | **Time (days)** | **Optical density of Shikonin derivatives*** | **Shikonin (mgg⁻¹)*** |
| 1 | 0 | 0 | 0 |
| 2 | 10 | 0.275 | 0.17 |
| 3 | 20 | 0.420 | 0.25 |
| 4 | 30 | 0.638 | 0.30 |
| 5 | 40 | 0.757 | 0.37 |
| 6 | 50 | 1.572 | 0.85 |

| | | | |
|---|---|---|---|
| *: - Mean of three replicates | | | |

The values refers to the increase in Shikonin Content (mg) of 1g of starting material.

## Claims

1. A method for the direct re-generation of Arnebia hispidissima plant said method comprising the steps of;
obtaining explants of Arnebia hispidissima species and sterilizing said explants,
subjecting said sterilized explants to the step of shoot generation by culturing the explants in shoot induction MS medium, wherein the shoot induction MS medium comprises Indole acetic acid (IAA) in the range of 0.01-2.0 mgl⁻¹, Kinetin in the range of 0.2-2.0 mgl⁻¹ and 6-Benzyl amino purine (BAP) in the range of 0.1-2.0 mgl⁻¹,
subjecting said shoots to the step of root generation by culturing said shoots in a root induction MS medium, comprising Indole butyric acid (IBA) in the range of 0.5 to 4.00 mgl⁻¹,
transferring said plantlets with shoots and root into the soil for regeneration.

2. The method as claimed in claim 1, wherein said explants are selected from a group consisting of shoot tips, leaf segments, nodal and internodal segments.

3. The method as claimed in claim 1, wherein the step of sterilizing is done using 0.1%HgCl₂ and then washing with distilled water to remove traces of HgCl₂.

4. A method for the production of callus and induction of shikonin comprising the steps of:
obtaining explants of Arnebia hispidissima,
sterilizing the nodal segment of said explants,
subjecting the sterilized nodal explant to the step of callus production by culturing in a callus induction MS medium comprising 0.5 mgl⁻¹ of Kinetin, 1.0 mgl⁻¹ 2,4-D dichlorophenoxy acetic acid and 100 mgl⁻¹of casein Hydrolysate CH,
culturing said callus in a shikonin induction MS medium comprising Kinetin in the range of 0.2-2.0 mgl⁻¹, 0.25 mgl ⁻¹ of BAP, 1.0 mgl⁻¹ of IAA and 100 mgl⁻¹ casein Hydrolysate (CH).

5. A method for genetic transformation in Arnebia hispidissima species by Agrobacterium rhizogenes for the production of Shikonin comprising the steps of:
sterilizing the explant of Arnebia hispidissima species,
inoculating the said sterilized explant in MS medium to form a clusterof multiple shoots, wherein said MS medium comprises (i) Kinetin in the range of 0.2-2.0 mgl⁻¹, 2.4-dichlorophenoxy acetic acid (2-4-D) in the range of 0.5-2.0 mgl⁻¹ and Casein Hydrolysate (CH) in the range of 50-150 mgl⁻¹, (ii) BAP in the range of 0.5-2.0 mgl⁻¹,2-4-D in the range 0.5-2.0 mgl⁻¹, and CH in the range 50-150 mgl⁻¹, (iii) Kinetin in the range of 0.2-2.0 mgl⁻¹, IAA in the range 0.01-2.0 mgl⁻¹ and CH in the range 50-150 mgl⁻¹, or (iv) BAP in the range of 0.1-2.0 mgl⁻¹, IAA in the range of 0.01-2.0 mgl⁻¹ and CH in the range of 50-150 mgl⁻¹.
preparing LB medium for Agrobacterium culture to obtain single colonies of Agrobacterium rhizogenes,
inoculating said colonies of Agrobacterium rhizogenes in LB medium in incubator,
infecting said shoots of Arnebia hispidissima species with Agrobacterium rhizogenes,
co-cultivating said infected explants in MS medium, wherein said MS medium is semi-solid and supplemented with IBA 0.5-2 mgL⁻¹ and 100 µM acetosyringone and said MS medium comprises (i) Kinetin in the range of 0.2-2.0 mgl⁻¹ IAA in the range of 0.01-2.0 mgl⁻¹ and CH in the range of 50-150 mgl⁻¹or (ii) BAP in the range of 0.1-2.0 mgl⁻¹ , IAA in the range of 0.01-2.0 mgl⁻¹, and CH in the range of 50-150 mgl⁻¹.
subjecting the said cultures to the step of incubation in complete darkness,
treating the infected shoots in a medium supplemented with antibiotic,
subjecting the said shoots to the step of sub culturing in root culture medium, and
extracting Shikonin derivatives from the roots.

6. The method as claimed in claim 5, wherein said LB medium is prepared by dissolving all the components of Luria Broth media in distilled water, the pH is adjusted to 7 with NaOH solution and Agar added after melting it and autoclaved at 15 psi at 121°C for 15 to 20 minutes.

7. The method as claimed in claim 5 wherein said antibiotic used is 250 mgl⁻¹ Cefotaxime.

8. The method as claimed in claim 5, wherein the explant derived from shoot tip, nodal, internodal and leaf segments were pre-cultured for three weeks at low light intensity on MS medium with acetosyringone 100 micro molar at 25 +/-2°C.

9. The method as claimed in claim 5, wherein the root culture MS medium is free of ammonium ion for enhancing the induction of shikonin production in transformed hairy roots.

## Patentansprüche

1. Verfahren für die direkte Regenerierung der *Arnebia hispidissima-*Pflanze, wobei das Verfahren die folgenden Schritte umfasst:
Gewinnen von Explantaten von *Arnebia* hispidissima-Spezies und Sterilisieren der Explantate,
Aussetzen der sterilisierten Explantate dem Schritt der Sprossbildung durch Kultivieren der Explantate in MS-Medium zur Sprossinduktion, worin das MS-Medium zur Sprossinduktion Indolessigsäure (IAA) im Bereich von 0,01-2,0 mgl⁻¹, Kinetin im Bereich von 0,2-2,0 mgl⁻¹ und 6-Benzylaminopurin (BAP) im Bereich von 0,1-2,0 mgl⁻¹ umfasst,
Aussetzen der Sprosse dem Schritt der Wurzelbildung durch Kultivieren der Sprosse in einem MS-Medium zur Wurzelinduktion, umfassend Indolbuttersäure (IBA) im Bereich von 0,5 bis 4,00 mgl⁻¹,
Transferieren der Jungpflanzen mit Sprossen und Wurzel in den Boden zur Regenerierung.

2. Verfahren nach Anspruch 1, worin die Explantate aus der Gruppe ausgewählt sind, bestehend aus Sprossspitzen, Blattsegmenten, Nodien- und Internodiensegmenten.

3. Verfahren nach Anspruch 1, worin der Schritt des Sterilisierens unter Verwendung von 0,1 % HgCl₂ durchgeführt wird, und dann Waschen mit destilliertem Wasser zum Entfernen von HgCl₂-Spuren.

4. Verfahren zur Produktion von Kallus und Induktion von Shikonin, umfassend die folgenden Schritte:
Gewinnen von Explantaten von *Arnebia hispidissima,*
Sterilisieren des Nodiensegments der Explantate,
Aussetzen des sterilisierten Nodienexplantats dem Schritt der Kallusproduktion durch Kultivieren in einem MS-Medium zur Kallusinduktion, umfassend 0,5 mgl⁻¹ Kinetin, 1,0 mgl⁻¹ 2,4-D-Dichlorphenoxyessigsäure und 100 mgl⁻¹ Caseinhydrolysat(CH),
Kultivieren des Kallus in einem MS-Medium zur Shikonin-Induktion, umfassend Kinetin im Bereich von 0,2-2,0 mgl⁻¹, 0,25 mgl⁻¹ BAP, 1,0 mgl⁻¹ IAA und 100 mgl⁻¹ Caseinhydrolysat (CH).

5. Verfahren zur genetischen Transformation in *Arnebia hispidissima-*Spezies durch *Agrobacterium rhizogenes* zur Produktion von Shikonin, umfassend die folgenden Schritte:
Sterilisieren des Explantats von *Arnebia hispidissima-*Spezies,
Inokulieren des sterilisierten Explantats in MS-Medium zur Bildung eines Clusters aus multiplen Sprossen, worin das MS-Medium Folgendes umfasst: (i) Kinetin im Bereich von 0,2-2,0 mgl⁻¹, 2,4-Dichlorphenoxyessigsäure (2-4-D) im Bereich von 0,5-2,0 mgl⁻¹ und Caseinhydrolysat (CH) im Bereich von 50-150 mgl⁻¹, (ii) BAP im Bereich von 0,5-2,0 mgl⁻¹, 2-4-D im Bereich von 0,5-2,0 mgl⁻¹ und CH im Bereich von 50-150 mgl⁻¹, (iii) Kinetin im Bereich von 0,2-2,0 mgl⁻¹, IAA im Bereich von 0,01-2,0 mgl⁻¹ und CH im Bereich von 50-150 mgl⁻¹ oder (iv) BAP im Bereich von 0,1-2,0 mgl⁻¹, IAA im Bereich von 0,01-2,0 mgl⁻¹ und CH Bereich von 50-150 mgl⁻¹,
Herstellen des LB-Mediums für die Agrobacterium-Kultur zum Erhalt von Einzelkolonien von *Agrobacterium rhizogenes,*
Inokulieren der Kolonien von *Agrobacterium rhizogenes* in LB-Medium im Inkubator,
Infizieren der Sprosse von *Arnebia hispidissima-Spezies* mit *Agrobacterium rhizogenes,*
Cokultivieren der infizierten Explantate in MS-Medium, worin das MS-Medium halbfest und mit IBA (0,5-2,0 mgl⁻¹) und 100 µM Acetosyringon supplementiert ist und das MS-Medium Folgendes umfasst: (i) Kinetin im Bereich von 0,2-2,0 mgl⁻¹, IAA im Bereich von 0,01-2,0 mgl⁻¹ und CH im Bereich von 50-150 mgl⁻¹ oder (ii) BAP im Bereich von 0,1-2,0 mgl⁻¹, IAA im Bereich von 0,01-2,0 mgl⁻¹ und CH im Bereich von 50-150 mgl⁻¹,
Aussetzen der Kulturen dem Inkubationsschritt in vollkommener Dunkelheit,
Behandeln der infizierten Sprosse in einem Medium, das mit einem Antibiotikum supplementiert ist,
Aussetzen der Sprosse dem Schritt des Subkultivierens in Wurzelkulturmedium, und
Extrahieren von Shikonin-Derivaten aus den Wurzeln.

6. Verfahren nach Anspruch 5, worin das LB-Medium durch Auflösen aller Komponenten des Luria-Bouillonmediums in destilliertem Wasser hergestellt wird, der pH mit NaOH-Lösung auf 7 eingestellt wird und Agar nach seinem Schmelzen zugefügt und 15 bis 20 Minuten bei 15 psi bei 121 °C autoklaviert wird.

7. Verfahren nach Anspruch 5, worin das verwendete Antibiotikum 250 mgl⁻¹ Cefotaxim darstellt.

8. Verfahren nach Anspruch 5, worin die Explantate, die sich von den Sprossspitzen-, Nodien-, Internodien- und Blattsegmenten herleiteten, drei Wochen bei geringer Lichtintensität auf MS-Medium mit Acetosyringon (100 mikromolar) bei 25°C +/- 2 °C vorkultiviert wurden.

9. Verfahren nach Anspruch 5, worin das MS-Medium zur Wurzelkultur frei von Ammoniumionen zur Förderung der Induktion der Shikonin-Produktion in transformierten Haarwurzeln ist.

## Revendications

1. Méthode de régénération directe de la plante *Arnebia hispidissima,* ladite méthode comprenant les étapes qui consistent à :
obtenir des explants de l'espèce *Arnebia hispidissima* et stériliser lesdits explants,
soumettre lesdits explants stérilisés à l'étape de génération de pousses par culture des explants dans un milieu MS pour induction de pousses, où le milieu MS pour induction de pousses contient de 0,01 à 2,0 mg.l⁻¹ d'acide indolacétique (AIA), de 0,2 à 2,0 mg.l⁻¹ de kinétine et 0,1 à 2,0 mg.l⁻¹ de 6-benzyl-aminopurine (BAP),
soumettre lesdites pousses à l'étape de génération de racines par culture des dites pousses dans un milieu MS pour induction de racines qui contient de 0,5 à 4,00 mg.l⁻¹ d'acide indolbutyrique (AIB),
transférer lesdites plantules dotées de pousses et de racines dans le sol pour régénération.

2. Méthode selon la revendication 1, où lesdits explants sont sélectionnés dans le groupe consistant en des bourgeons caulinaires, segments foliaires et segments nodaux et internodaux.

3. Méthode selon la revendication 1, où l'étape de stérilisation est effectuée en utilisant de l'HgCl₂ à 0,1 % et est suivie d'un lavage à l'eau distillée pour éliminer les traces d'HgCl₂.

4. Méthode de formation de cals et d'induction de la production de shikonine, ladite méthode comprenant les étapes qui consistent à :
obtenir des explants d*'Arnebia hispidissima,*
stériliser le segment nodal desdits explants,
soumettre l'explant nodal stérilisé à l'étape de formation d'un cal par culture dans un milieu MS pour induction de cals qui contient 0,5 mg.l⁻¹ de kinétine, 1,0 mg.l-¹ d'acide 2,4-D-dichlorophénoxyacétique et 100 mg.l⁻¹ d'hydrolysat de caséine (HC),
cultiver lesdits cals dans un milieu MS pour induction de la production de shikonine qui contient de 0,2 à 2,0 mg.l⁻¹ de kinétine, 0,25 mg.l⁻¹ de BAP, 1,0 mg.l⁻¹ d'AIA et 100 mg.l⁻¹ d'hydrolysat de caséine (HC) .

5. Méthode de transformation génétique de l'espèce *Arnebia hispidissima* par *Agrobacterium rhizogenes* pour la production de shikonine, ladite méthode comprenant les étapes qui consistent à :
stériliser l'explant de l'espèce *Arnebia hispidissima,*
inoculer ledit explant stérilisé dans un milieu MS pour former un groupe de pousses multiples, où ledit milieu MS contient (i) de 0,2 à 2,0 mg.l⁻¹ de kinétine, de 0,5 à 2,0 mg.l⁻¹ d'acide 2,4-dichlorophénoxyacétique (2,4-D) et de 50 à 150 mg.l⁻¹ d'hydrolysat de caséine (HC), (ii) de 0,5 à 2,0 mg.l⁻¹ de BAP, de 0,5 à 2,0 mg.l⁻¹ de 2,4-D et de 50 à 150 mg.l⁻¹ d' HC, (iii) de 0,2 à 2,0 mg.l⁻¹ de kinétine, de 0,01 à 2,0 mg.l⁻¹ d'AIA et de 50 à 150 mg.l⁻¹ d' HC, ou (iv) de 0,1 à 2,0 mg.l⁻¹ de BAP, de 0,01 à 2,0 mg.1⁻¹ d'AIA et de 50 à 150 mg.l⁻¹ d'HC,
préparer un milieu LB de culture d'*Agrobacterium* pour obtenir des colonies isolées d*'Agrobacterium rhizogenes,*
inoculer un milieu LB avec lesdites colonies d*'Agrobacterium rhizogenes* dans un incubateur,
infecter lesdites pousses de l'espèce *Arnebia hispidissima* avec *Agrobacterium rhizogenes,*
co-cultiver lesdits explants infectés dans un milieu MS, où ledit milieu MS est un milieu semi-solide supplémenté par 0,5 à 2,0 mg.l⁻¹ d'AIB et 100 µM d'acétosyringone qui comprend (i) de 0,2 à 2,0 mg.l⁻¹ de kinétine, de 0,01 à 2,0 mg.l⁻¹ d'AIA et de 50 à 150 mg.l⁻¹ d' HC, ou (ii) de 0,1 à 2,0 mg.l⁻¹ de BAP, de 0,01 à 2,0 mg.l⁻¹ d'AIA et de 50 à 150 mg.l⁻¹ d'HC,
soumettre lesdites cultures à une étape d'incubation à l'obscurité totale,
traiter les pousses infectées dans un milieu supplémenté par un antibiotique,
soumettre lesdites pousses à une étape de repiquage dans un milieu d'induction de racines et
extraire les dérivés de la shikonine des racines.

6. Méthode selon la revendication 5, où ledit milieu LB est préparé en dissolvant tous les composants du milieu Luria Broth dans de l'eau distillée, en ajustant le pH à 7 avec une solution de NaOH et en ajoutant la gélose après l'avoir fait fondre et soumise à un autoclavage à 15 psi et 121°C pendant 15 à 20 minutes.

7. Méthode selon la revendication 5, où ledit antibiotique est le céfotaxime à 250 mg.l⁻¹.

8. Méthode selon la revendication 5, où les explants dérivés de bourgeons caulinaires et de segments foliaires, nodaux et internodaux ont été pré-cultivés pendant trois semaines à une intensité lumineuse basse et à 25 +/- 2 °C sur un milieu MS contenant de l'acétosyringone 100 µM.

9. Méthode selon la revendication 5, où le milieu MS pour induction de racines est exempt d'ions ammonium pour stimuler l'induction de la production de shikonine dans les racines chevelues transformées.
